# EUROPEAN PATENT SPECIFICATION

(11) **EP 2 740 439 B1**
(45) Date of publication and mention of the grant of the patent: **31.10.2018**
(21) Application number: 12196266.6
(22) Date of filing: 10.12.2012
(51) Int. Cl.: A61F 2/07

(54) **Stent graft having two coaxial stents and a graft tissue**
Stentgraft mit zwei koaxialen Stents und Gewebetransplantat
Endoprothèse comportant deux extenseurs coaxiaux et tissu greffé

(43) Date of publication of application: 11.06.2014
(73) Proprietor: Abbott Laboratories Vascular Enterprises Limited, Terrace, Dublin 2 (IE)
(72) Inventor: Grandt, Axel, 72479 Straßberg (DE)
(74) Representative: Peters, Hajo

(56) References cited:
- EP-A1- 0 878 173
- WO-A1-2004/047687
- US-A- 5 916 264
- US-A1- 2006 259 131

## Description

The invention relates to a stent graft as claimed, having an inner expandable stent and an outer expandable stent with a graft tissue held in between.

### Background of the invention

In medicine, a natural conduit in a body may be locally flow constricted or damaged. A stent graft may be inserted into this natural conduit, in order to counteract such a flow constriction or to repair damage. A stent graft is an artificial tube-like device having a stent with meshed walls, the dimension of the stent, in particular the diameter, being expandable once it is positioned in the natural conduit appropriately. In the expanded shape, the stent holds the natural conduit open to allow the flow of body fluids or the access for surgery. The stent is covered with a tubular fabric or graft material which is supported by the stent. This way the graft material can close a damaged spot of the natural conduit.

Stent grafts are known having two coaxially arranged stents with a graft tissue arranged in between them, see for example US5916264. As usually the inner and outer stents have the same or similar wire patterns, defects in the tissue graft may be created due to the interaction of the inner and outer stent. Due to the wires of the two stents being positioned on both sides of the same spot of the tissue graft, the tissue graft may be subjected increased load at these spots.

US2006259131 discloses medical devices, such as endoprostheses, and methods of making the devices. The medical devices can include a composite cover formed of a deposited metallic film and one or more polymer layers. The polymer layers contribute to mechanical or biological properties of the endoprosthesis.

### Summary of the invention

It is an object of the present invention to decrease load on a tissue graft used between two stents.

This object is solved with a stent graft according to the independent claim. Advantageous further developments are subject of the dependent claims.

According to an embodiment of the invention, a stent graft is provided, comprising an inner expandable stent having a tubular wall, and an outer expandable stent having a tubular wall and being arranged coaxial around the inner stent, the outer stent having a different length as the inner stent in a state of equal stent expansion. Further, the stent graft comprises a tissue graft disposed coaxial to and entirely in between the inner and outer stent, the tissue graft has the same length as the shorter one of the inner and outer stent, in particular in a state, in which the tissue graft and the shorter stent is in a non-expanded state or in other words in a state as the stent graft is delivered from the manufacturer, wherein the longitudinal centers of the outer stent and the tissue graft are aligned with the longitudinal center of the inner stent. In particular, the outer stent is the shorter one of the inner and outer stent. The advantage of this embodiment is that the different length of the two stents allows offsetting the stents from each other. Thus, also offsetting their wire patterns, which reduces the area of direct overlap of the stent wires, hence reduces the load on the tissue graft and also reduces the risk of causing defects in the tissue graft.

According to a further embodiment of the invention, the shorter one of the inner and outer stent is of an open cell design. The term "open cell design" is well known to a person skilled in the art. The benefit of this embodiment is an increased flexibility of the stent graft.

According to a further embodiment of the invention, the longer one of the inner and outer stent is of a closed cell design at its longitudinal end portions, and of an open cell design in between the end portions. The term "closed cell design" is also well known to a person skilled in the art. The advantage of this embodiment is that the portion of the stent graft, where two stents are present, can be designed more flexible, and the portion, where there are only the end portions of a single stent, the stent graft is provided with an increased stability due to the closed cell design.

According to a further embodiment of the invention, the closed cell design at the longitudinal ends is formed by circumferentially arranging rhombic wire segments, adjacent ones of which are directly connected to each other at a corner of the rhombic shape.

According to a further embodiment of the invention, the open cell design is formed by arranging rhombic wire segments, adjacent ones of which are directly connectable to each other at a corner of the rhombic shape or via tie bars connecting the corners of adjacent rhombic wire segments, wherein each corner of each rhombic wire segment which corner is facing towards an adjacent rhombic wire segment is a possible connecting point, and at least some possible connecting points are not used for connecting.

According to a further embodiment of the invention, the closed cell design at the longitudinal ends is formed by circumferentially extending ring members having a serpentine shape, wherein two adjacent ring members are directly connected by connecting peaks of the serpentine shape of one ring member with valleys of the serpentine shape of an adjacent ring member.

According to a further embodiment of the invention, the open cell design is formed by circumferentially extending ring members having a serpentine shape, adjacent ones of which are connectable via tie bars connecting possible connecting points, wherein each peak or valley of the serpentine shape, which peak or valley faces towards an adjacent ring member is a possible connecting point, and at least some possible connecting points are not used for connecting.

According to a yet further embodiment of the invention, the tubular walls of the inner and outer stent comprise a plurality of wire segments, each wire segment having a peripheral area of the tubular wall, which peripheral area is bordered by the wires framing this peripheral area.

According to a further embodiment of the invention, a pattern (with which the wires are arranged) of the tubular wall of the inner stent is offset from a pattern (with which the wires are arranged) of the tubular wall of the outer stent.

According to a further embodiment of the invention, at both ends of the shorter one of the inner and outer stent, half of the outermost wire segments of the longer one of the inner and outer stent project out of the shorter stent.

According to further embodiment of the invention, the inner and outer stent are longitudinally offset from each other by half the longitudinal dimension of the outermost wire segments of the longer one of the inner and outer stent.

According to a yet further embodiment of the invention, a wire segment density per peripheral surface area of the tubular wall, is higher at longitudinal end portions of the longer one of the inner and outer stent than in between these end portions.

According to a further embodiment of the invention, the inner and outer stent are balloon-expandable stents.

These and other embodiments are described in more detail with reference to the Figures.

### Brief description of the Figures

Fig. 1 schematically shows a stent graft according to an exemplary embodiment of the invention;
Fig. 2 schematically shows another exemplary embodiment of a stent, in which embodiment the inner and outer stents are constructed differently, and
Fig. 3 illustrates the offset between the inner and outer stent of the stent graft.

### Detailed description of exemplary embodiments of the invention

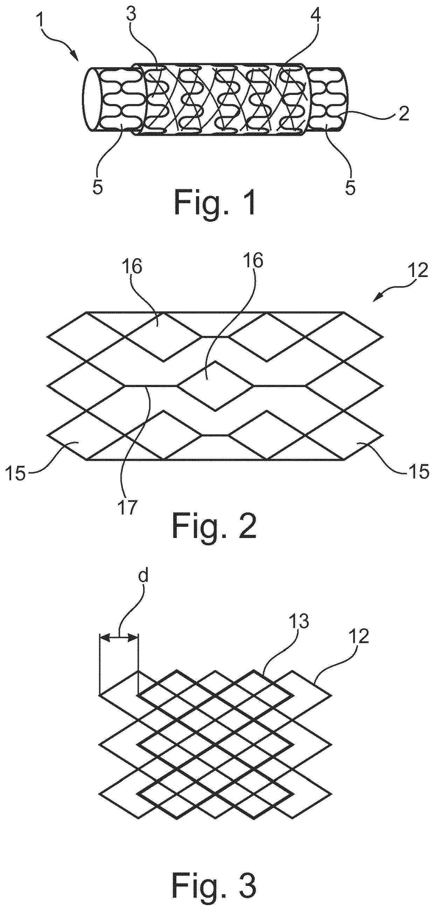

Fig. 1 shows the stent graft 1 according to an exemplary embodiment of the invention. The stent graft 1 comprises an inner stent 2 having a tubular shape formed by tubular walls, and an outer stent 3 which also has a tubular shape formed by tubular walls. Both stents 2, 3 are made of biocompatible material. The two stents 2, 3 are coaxially arranged such that the outer stent 3 surrounds the inner stent 2 such that their longitudinal centers are aligned with each other. In this embodiment, the outer stent 3 is shorter than the inner stent 2, such that equally long portions of the inner stent 2 project out of the longitudinal ends of the outer stent 3. As the stents 2, 3 might change their length slightly due to foreshortening (a slight reduction in length during radial expansion), the length of both stents 2, 3 shall be compared with each other in a state in which both are expanded to an equal degree. Alternatively to the above, also the outer stent 3 may be longer than the inner stent 2 by the same longitudinal extend.

As well known from the state of the art, the stents are constructed of a wire arrangement, wherein the wires extend within a peripheral surface of the tubular shape in certain patterns.

For example the stents can be constructed in a serpentine pattern, as indicated in Fig. 1. In this design, the stents are constructed as a plurality of circumferentially extending ring members, each of which is in the form of serpentines which extend within the peripheral surface of the tubular wall. The ring members are connected with substantially longitudinally extending tie bars. When orientating the stents upright such that the longitudinal axis is vertical, the serpentine-shaped ring members show peaks and valleys. The longitudinal tie bars can be attached to a valley of a ring member and extend to the valley of the consecutive ring member, and are attached to a peak of a ring member and extend to the peak of a consecutive ring member. If all these connections are realized, the design is referred to as a closed cell design, if some or all tie bars are omitted, the design is referred to as an open cell design. A closed cell design can also be realized by connecting each of the peaks of a ring member directly with respective valleys of the adjacent ring member. This later mentioned closed cell structure is for example utilized at the longitudinal end portions of the inner stent 2, shown in Fig. 1. The two outermost ring members on each longitudinal end are connected such that the peaks of one ring member are directly connected to the valleys of the adjacent ring member. The peripheral surface area in between two adjacent connection points, which surface area is framed by the connected wires of the ring members, is referred to as wire segment 5. This way, the outermost wire segments form a closed ring which forms the closed cell design at the longitudinal ends. In Fig. 1, only two of the six illustrated outermost wire segments 5 of the inner stent 2 are provided with a reference sign for simplicity reasons. Thus, in the embodiment of Fig. 1, the inner stent is projecting out of each end of the outer stent 2 by the longitudinal length of such formed outermost wire segments 5 of the inner stent 3. In between the outermost segments 5 formed this way (the closed cell design), the inner stent 2 is provided with the above described open cell design using tie bars for connecting the ring elements, in which some or all of the longitudinal tie bars are left out. In Fig. 1 this part of the design is covered by the overlaying elements (such as the tissue graft).

In between the inner stent 2 and the outer stent 3, a tissue graft 4 is arranged. The tissue graft 4 is illustrated with hatched diagonal lines. The tissue graft 4 has the same length and longitudinal arrangement as the shorter one out of the inner stent 2 and outer stent 3, which is in the case of Fig. 1 the outer stent 3. The tissue graft 4 or membrane can be biological tissue such as a mammary vein or other veins of an animal. Also a biological tissue other than a vein can be used for the tissue graft. Moreover, the tissue graft can be made of suitable synthetic polymers which are biocompatible to the human body.

In the following a further exemplary embodiment is described, wherein only its differences to the above description are mentioned, apart from these differences, the above description shall also apply to the following exemplary embodiment.

Fig. 2 shows another exemplary embodiment in which the inner and outer stents are constructed differently. According to this embodiment, the stents are constructed of a wire arrangement, wherein the wires extend within a peripheral surface of the tubular shape in a rhombic pattern instead of a serpentine pattern. In this design, the stents are constructed as a plurality of rhombic segments 15, 16 which are either directly connected to each other at one of their corners or which are connected to each other via a tie bar 17 which is attached to respective corners of the connected rhombic segments 15, 16. Fig. 2 shows in particular an inner stent 12 having a closed cell design at its longitudinal end portions and a closed cell design in between these end portions. These end portions are formed by attaching a plurality of rhombic segments directly to each other by connecting them at a corner of the rhombic shape, respectively. This way a closed ring of adjoining rhombic segments is formed. In between, these longitudinal end portions, the inner stent 12 is provided with an open cell design, which is realized with a plurality of connected rhombic segments, wherein compared to the closed cell design, some rhombic segments might be left out (the density of rhombic segments per peripheral surface area is smaller in the open cell design than in the closed cell design) and/or by connecting adjacent rhombic segments via tie bars 17, instead of direct connections and leaving out some of the tie bars 17. The not illustrated outer stent is entirely constructed in the open cell design in this embodiment.

The different length of the inner and outer stent of the above embodiments allows offsetting the design patterns (the serpentine pattern and/or the rhombic pattern) as indicated in Fig. 3. Even a rhombic design is illustrated in Fig. 3, this also applies to the serpentine design of Fig. 1. Fig. 3 shall only illustrate the offset and does not show the provided open cell pattern. As indicated in Fig. 3, the inner stent is by the length of the outermost segments longer in length, which allows offsetting the wire segments of the inner stent from the wire segments of the outer stent by half of the length of an outermost wire segment length, as indicated by reference sign d. This way the contacting area of the wires of the inner stent with the wires of the outer stent are minimized, which is advantageous for the durability of the graft tissue arranged in between.

While the invention has been illustrated and described in detail in the drawings and foregoing description, such illustration and description are to be considered illustrative or exemplary and not restrictive and it is not intended to limit the invention to the disclosed embodiments. The mere fact that certain measures are recited in mutually different dependent claims does not indicate that a combination of these measures cannot be used advantageously.

## Claims

1. Stent graft (1) comprising
an inner expandable stent (2; 12) having a tubular wall;
an outer expandable stent (3; 13) having a tubular wall and being arranged coaxial around the inner stent (2; 12), the outer stent (3; 13) having a different length as the inner stent (2; 12) in a state of equal stent expansion;
a tissue graft (4) disposed coaxial to and entirely in between the inner and outer stent (2, 3; 12, 13), the tissue graft (4) having the same length as the shorter one of the inner and outer stent, wherein the longitudinal centers of the outer stent (3; 13) and the tissue graft (4) are aligned with the longitudinal center of the inner stent (2; 12).

2. Stent graft (1) according to claim 1, wherein the shorter one of the inner and outer stent (2, 3; 12, 13) is of an open cell design.

3. Stent graft (1) according to one of the preceding claims, wherein the longer one of the inner and outer stent (2, 3; 12, 13) is of a closed cell design at its longitudinal end portions, and of an open cell design in between the end portions.

4. Stent graft (1) according to claim 3, wherein the closed cell design at the longitudinal ends is formed by circumferentially arranging rhombic wire segments (15), adjacent ones of which are directly connected to each other at a corner of the rhombic shape.

5. Stent graft (1) according to one of claims 2 to 4, wherein the open cell design is formed by arranging rhombic wire segments (16), adjacent ones of which are directly connectable to each other at a corner of the rhombic shape or via tie bars (17) connecting the corners of adjacent rhombic wire segments (16), wherein each corner of each rhombic wire segment (16) which corner is facing towards an adjacent rhombic wire segment (16) is a possible connecting point, and at least some possible connecting points are not used for connecting.

6. Stent graft (1) according to claim 3, wherein the closed cell design at the longitudinal ends is formed by circumferentially extending ring members having a serpentine shape, wherein two adjacent ring members are directly connected by connecting peaks of the serpentine shape of one ring member with valleys of the serpentine shape of an adjacent ring member.

7. Stent graft (1) according to one of claims 2, 3 or 6, wherein the open cell design is formed by circumferentially extending ring members having a serpentine shape, adjacent ones of which are connectable via tie bars connecting possible connecting points, wherein each peak or valley of the serpentine shape, which peak or valley faces towards an adjacent ring member is a possible connecting point, and at least some possible connecting points are not used for connecting.

8. Stent graft (1) according to one of the preceding claims, wherein the tubular walls of the inner and outer stent (2, 3; 12, 13) comprise a plurality of wire segments (5; 15, 16), each wire segment (5; 15, 16) having a peripheral area of the tubular wall, which peripheral area is bordered by the wires framing this peripheral area.

9. Stent graft (1) according to one of the preceding claims, wherein a pattern of the tubular wall of the inner stent (2; 12) is offset from a pattern of the tubular wall of the outer stent (3; 13).

10. Stent graft (1) according to claim 8, wherein at both ends of the shorter one of the inner and outer stent (2, 3; 12, 13), half of the outermost wire segments (5; 15) of the longer one of the inner and outer stent project out of the shorter stent.

11. Stent graft (1) according to one of claims 8 to 10, wherein the inner and outer stent (2, 3; 12, 13) are longitudinally offset from each other by half the longitudinal dimension (d) of the outermost wire segments (5; 15) of the longer one of the inner and outer stent.

12. Stent graft (1) according to one of claims 8 to 11, a wire segment density per peripheral surface area of the tubular wall, is higher at longitudinal end portions of the longer one of the inner and outer stent than in between these end portions.

13. Stent graft (1) according to one of the preceding claims, wherein the inner and outer stent (2, 3; 12, 13) are balloon-expandable stents.

## Patentansprüche

1. Stentgraft (1) umfassend
einen inneren expandierbaren (2; 12) Stent, der eine rohrförmige Wand aufweist;
einen äußeren expandierbaren Stent (3; 13), der eine rohrförmige Wand aufweist und koaxial um den inneren Stent (2; 12) herum angeordnet ist, wobei der äußere Stent (3; 13) eine andere Länge als der innere Stent (2; 12) in einem Zustand der gleichen Stentexpansion aufweist;
ein Gewebetransplantat (4), das koaxial an und vollständig zwischen dem inneren und äußeren Stent (2, 3; 12, 13) angebracht ist, wobei das Gewebetransplantat (4) die gleiche Länge wie der kürzere des inneren und äußeren Stents aufweist, wobei die Längsmitten des äußeren Stents (3; 13) und des Gewebetransplantats (4) auf die Längsmitte des inneren Stents (2; 12) ausgerichtet sind.

2. Stentgraft (1) nach Anspruch 1, wobei der kürzere des inneren und äußeren Stents (2, 3; 12, 13) eine offenzellige Gestalt besitzt.

3. Stentgraft (1) nach einem der vorhergehenden Ansprüche, wobei der längere des inneren und äußeren Stents (2, 3; 12, 13) an seinen Längsendabschnitten eine geschlossenzellige Gestalt und zwischen den Endabschnitten eine offenzellige Gestalt besitzt.

4. Stentgraft (1) nach Anspruch 3, wobei die geschlossenzellige Gestalt an den Längsenden durch die umfängliche Anordnung rhombischer Drahtsegmente (15) gebildet ist, von denen benachbarte an einer Ecke der rhombischen Form direkt miteinander verbunden sind.

5. Stentgraft (1) nach einem der Ansprüche 2 bis 4, wobei die offenzellige Gestalt durch Anordnung rhombischer Drahtsegmente (16) gebildet ist, von denen benachbarte an einer Ecke der rhombischen Form oder über die Ecken von benachbarte rhombische Drahtsegmente (16) verbindenden Zugstäben (17) direkt miteinander verbindbar sind, wobei jede Ecke jedes rhombischen Drahtsegments (16), welche Ecke einem benachbarten rhombischen Drahtsegment (16) zugewandt ist, ein möglicher Verbindungspunkt ist, und mindestens einige mögliche Verbindungspunkte nicht zum Verbinden verwendet werden.

6. Stentgraft (1) nach Anspruch 3, wobei die geschlossenzellige Gestalt an den Längsenden durch sich umfänglich erstreckende Ringelemente gebildet ist, die eine Serpentinenform aufweisen, wobei zwei benachbarte Ringelemente durch das Verbinden von Spitzen der Serpentinenform eines Ringelements mit Tälern der Serpentinenform eines benachbarten Ringelements direkt verbunden sind.

7. Stentgraft (1) nach einem der Ansprüche 2, 3 oder 6, wobei die offenzellige Gestalt durch sich umfänglich erstreckende Ringelemente gebildet ist, die eine Serpentinenform aufweisen, von denen benachbarte über mögliche Verbindungspunkte verbindende Zugstäbe verbindbar sind, wobei jede Spitze oder jedes Tal der Serpentinenform, welche Spitze oder welches Tal einem benachbarten Ringelement zugewandt ist, ein möglicher Verbindungspunkt ist, und mindestens einige mögliche Verbindungspunkte nicht zum Verbinden verwendet werden.

8. Stentgraft (1) nach einem der vorhergehenden Ansprüche, wobei die rohrförmigen Wände des inneren und äußeren Stents (2, 3; 12, 13) eine Vielzahl von Drahtsegmenten (5; 15, 16) umfassen, wobei jedes Drahtsegment (5; 15, 16) eine Umfangsfläche der rohrförmigen Wand aufweist, welche Umfangsfläche durch die diese Umfangsfläche umrahmenden Drähte begrenzt ist.

9. Stentgraft (1) nach einem der vorhergehenden Ansprüche, wobei ein Muster der rohrförmigen Wand des inneren Stents (2; 12) zu einem Muster der rohrförmigen Wand des äußeren Stents (3; 13) versetzt ist.

10. Stentgraft (1) nach Anspruch 8, wobei an beiden Enden des kürzeren des inneren und äußeren Stents (2, 3; 12, 13) die Hälfte der äußersten Drahtsegmente (5; 15) des längeren des inneren und äußeren Stents aus dem kürzeren Stent herausragt.

11. Stentgraft (1) nach einem der Ansprüche 8 bis 10, wobei der innere und äußere Stent (2, 3; 12, 13) um die Hälfte der Längsabmessung (d) der äußersten Drahtsegmente (5; 15) des längeren des inneren und äußeren Stents in Längsrichtung voneinander versetzt sind.

12. Stentgraft (1) nach einem der Ansprüche 8 bis 11, wobei eine Drahtsegmentdichte pro Umfangsflächenbereich der rohrförmigen Wand höher an Längsendabschnitten des längeren des inneren und äußeren Stents als zwischen diesen Endabschnitten ist.

13. Stentgraft (1) nach einem der vorhergehenden Ansprüche, wobei der innere und äußere Stent (2, 3; 12, 13) ballonexpandierbare Stents sind.

## Revendications

1. Endoprothèse (1) comprenant
un stent interne expansible (2; 12) ayant une paroi tubulaire ;
un stent externe expansible (3; 13) ayant une paroi tubulaire et étant agencé coaxialement autour du stent interne (2; 12), le stent externe (3; 13) ayant une longueur différente du stent interne (2; 12) dans un état d'expansion égale de stent ;
une greffe de tissu (4) disposée de manière coaxiale à et entièrement entre les stents interne et externe (2, 3; 12, 13), la greffe de tissu (4) ayant la même longueur que le plus court du stent interne et externe, les centres longitudinaux du stent externe (3; 13) et de la greffe de tissu (4) étant alignés avec le centre longitudinal du stent interne (2; 12).

2. Endoprothèse (1) selon la revendication 1, dans laquelle le plus court des stents interne et externe (2, 3; 12, 13) présente une conception à cellules ouvertes.

3. Endoprothèse (1) selon l'une des revendications précédentes, dans laquelle le plus long des stents interne et externe (2, 3; 12, 13) présente une conception à cellules fermées au niveau de ses parties d'extrémité longitudinales et une conception à cellules ouvertes entre les parties d'extrémité.

4. Endoprothèse (1) selon la revendication 3, dans laquelle la conception à cellules fermées aux extrémités longitudinales est formée par des segments de fil rhombiques (15) disposés de manière circonférentielle, dont des éléments adjacents sont directement reliés l'un à l'autre au niveau d'un coin de la forme rhombique

5. Endoprothèse (1) selon l'une des revendications 2 à 4, dans laquelle la conception à cellules ouvertes est formée par l'agencement de segments de fil rhombiques (16), dont des éléments adjacents peuvent être directement reliés l'un à l'autre au niveau d'un coin de la forme rhombique ou par l'intermédiaire de barres d'ancrage (17) reliant les coins de segments de fils rhombiques adjacents (16), chaque coin de chaque segment de fil rhombique (16) orienté vers un segment de fil rhombique adjacent (16) constituant un point de connexion possible et au moins certains points de connexion possibles ne sont pas utilisés pour la connexion.

6. Endoprothèse (1) selon la revendication 3, dans laquelle la conception à cellules fermées aux extrémités longitudinales est formée par des éléments d'anneau s'étendant circonférentiellement ayant une forme en serpentin, deux éléments d'anneau adjacents étant directement reliés par des sommets de connexion de forme en serpentin d'un élément d'anneau avec des vallées en forme de serpentin d'un élément d'anneau adjacent.

7. Endoprothèse (1) selon l'une des revendications 2, 3 ou 6, dans laquelle la conception à cellules ouvertes est formée par des éléments d'anneau s'étendant circonférentiellement en forme de serpentin dont des éléments adjacents peuvent être reliés par des barres d'ancrage reliant des points de connexion possibles, chaque sommet ou vallée de la forme en serpentin, ledit sommet ou ladite vallée étant tourné(e) vers un élément d'anneau adjacent est un point de connexion possible, et au moins certains points de connexion possibles ne sont pas utilisés pour la connexion.

8. Endoprothèse (1) selon l'une des revendications précédentes, dans laquelle les parois tubulaires des stents interne et externe (2, 3; 12, 13) comprennent une pluralité de segments de fil (5; 15, 16), chaque segment de fil (5; 15, 16) ayant une zone périphérique de la paroi tubulaire, ladite zone périphérique étant bordée par les fils encadrant cette zone périphérique.

9. Endoprothèse (1) selon l'une des revendications précédentes, dans laquelle un motif de la paroi tubulaire du stent interne (2; 12) est décalé par rapport à un motif de la paroi tubulaire du stent externe (3; 13).

10. Endoprothèse (1) selon la revendication 8, dans laquelle, aux deux extrémités du stent interne et externe plus court (2, 3; 12, 13), la moitié des segments de fil les plus à l'extérieur (5; 15) du plus long des stents intérieur et extérieur fait saillie à partir du stent plus court.

11. Endoprothèse (1) selon l'une des revendications 8 à 10, dans laquelle les stents interne et externe (2, 3; 12, 13) sont décalés longitudinalement les uns des autres de la moitié de la dimension longitudinale (d) des segments de fil les plus extérieurs (5; 15) du plus long des stents interne et externe.

12. Endoprothèse (1) selon l'une des revendications 8 à 11, dans laquelle une densité de segment de fil par surface périphérique de la paroi tubulaire étant plus élevée dans les parties d'extrémité longitudinales du plus long du stent interne et externe qu'entre ces parties d'extrémité.

13. Endoprothèse (1) selon l'une des revendications précédentes, dans laquelle les stents interne et externe (2, 3; 12, 13) sont des stents expansibles par ballonnet.
